# EUROPEAN PATENT APPLICATION

(11) **EP 2 672 410 A1**
(43) Date of publication of application: **11.12.2013**
(21) Application number: 12170783.0
(22) Date of filing: 05.06.2012
(51) Int. Cl.: G06F 19/00

(54) **Context based software configuration**

(71) Applicant: Agfa Healthcare, 2640 Mortsel (BE)
(72) Inventor: Felix, Joost, 2640 Mortsel (BE); Kieckens, Wannes, 2640 Mortsel (BE); De Paepe, Nadia, 2640 Mortsel (BE)

(57) **Abstract**

The present invention is directed towards the configuration of a software application by assigning configuration values sets to context of use which is composed of different levels with different priority scores. During runtime the best matching configuration values set is chosen according to the run-time context of use and the associated priorities.

## Description

### FIELD OF THE INVENTION

The present invention relates to a method for configuration of a software application, such as a computer program for medical report generation.

### BACKGROUND OF THE INVENTION

Software applications used by a large number of users with a large number of specific, individual demands are preferably adapted to these user demands.

For example when a radiologist in a radiology department of a hospital writes a report on a certain study (or examination), he runs a report generating software and should choose a specific report structure to be used from a large number of pre-configured report structures. These different report structures are intended for different facilities, departments, modality types, could also for different radiologists' individual preferences.

Preferably he chooses a specific report structure that matches the study dealt with in the report. However, since there are many types of studies, many types of report structures would be required. Each type of report to be generated by the same software could be treated as an individual case and the software could be configured for each individual case. However, this would result in a time consuming and very elaborate job.

In a hospital environment the same problem occurs with regard to the implementation of other applications, e.g. in case of software implementing a hanging protocol to be used when displaying different images of a study or with normal text or macro report generation or with the generation of a procedure plan etc.

In general, a need for configuration for specific needs exists with all software programs that can be used in a complex environment by many users with specific desires.

US 7, 831, 960 discloses a method for configuration of a program in which the program can obtain values for its configuration variables during execution.

This patent deals with the situation in which multiple conflicting sources (places to store the values for the configuration parameters, configuration levels) exist for the values of a configuration variable. According to the disclosure, a single priority semantic is set for all configuration variables. Such a priority semantic specifies an ordering of the values of the configuration variables. The semantic is hierarchical and defines a priority based on the configuration level. The nearer a value is to the user of the program, the more priority exists for using the value. So, for example a value set in a command line will typically be given a higher priority than a default value. Thus if a command line value exists, the command line value will be used regardless of whether a default value exists.

A drawback of this prior art method is that the configuration is not stored in a central repository, e.g. in a configuration file, system registry, environment variables, command lines, etc. It is not convenient for the users.

It is an aspect of the present invention to provide a method for configuration of a software program for different users of the same functionality.

### SUMMARY OF THE INVENTION

The above-mentioned aspects are realized by a method having the specific steps set out in claim 1. Specific features for preferred embodiments of the invention are set out in the dependent claims. According to the present invention, the configuration of a software application is performed during the run time on the basis of the context of use.

In the present invention 'software application', 'software program', 'computer program' and 'software-implemented application' are used as synomyms.

In the present invention 'context of use' is defined as an identification of the environment in which the software is run. Context of use may be identified by one of several possible items such as a user, a user group, a department in which the software is used, a workstation on which the software is used etc.). It may also be identified by a code referring to such identifying items.

In medical applications this 'context of use' can be an identification of the user (name of a doctor, nurse, technician ...), a department in a hospital (e.g. radiology, oncology, pediatrics ...), a modality used for generating a medical image, a workstation identification, a hardware system running the software etc.

In one embodiment the context of use is composed of different levels with different priority. For example for a runtime context giving information on the user's name, the department he is working in, the facility where the department he is working in is situated, the user group he belongs to, the workstation he is working on, the workstation group, the medical procedure/study he is dealing with, and finally the entire system. Runtime context may consist of some of these items or of all of them. Different levels may be assigned to these items and different hierarchically ordered priority scores may be allocated to these different levels.

Context of use and context of use levels with corresponding priority scores as well as the corresponding sets of configuration values for the software application are stored in advance.

In the runtime, the runtime context is first determined. Then, the configuration that matches the runtime context is searched for and retrieved from the repository where these data were stored in advance and applied. If a matching configuration is not found, a higher level is searched for and the configuration settings of that higher level are applied. If the level is already the highest level, default settings are applied. The hierarchy of configuration levels ensures that the most relevant settings according to the runtime can be found and applied.

Preferably the method provides that certain settings can be overruled.

The embodiments of the methods of the present invention are generally implemented in the form of a computer program product adapted to carry out the method steps of the present invention when run on a computer. The computer program product is commonly stored in a computer readable carrier medium such as a CD-ROM.

Alternatively the computer program product takes the form of an electric signal and can be communicated to a user through electronic communication.

Further advantages and embodiments of the present invention will become apparent from the following description and drawings.

### BRIEF DESCRIPTION OF THE DRAWINGS

Fig. 1 shows a flow chart illustrating the method of the present invention in general,
Fig. 2 illustrates the configuration selection based on preset priorities.

### DETAILED DESCRIPTION OF THE INVENTION

The present invention has been developed in the context of a hospital environment for software applications that are used by multiple departments and users, but is not limited to this application and can be applied to other computer programs that need configuration for different situations or users.

Examples of such software applications in a hospital environment are report generation, software controlling the visualization of images using hanging protocols etc, reporting printing, text macro, procedure plan initializing.

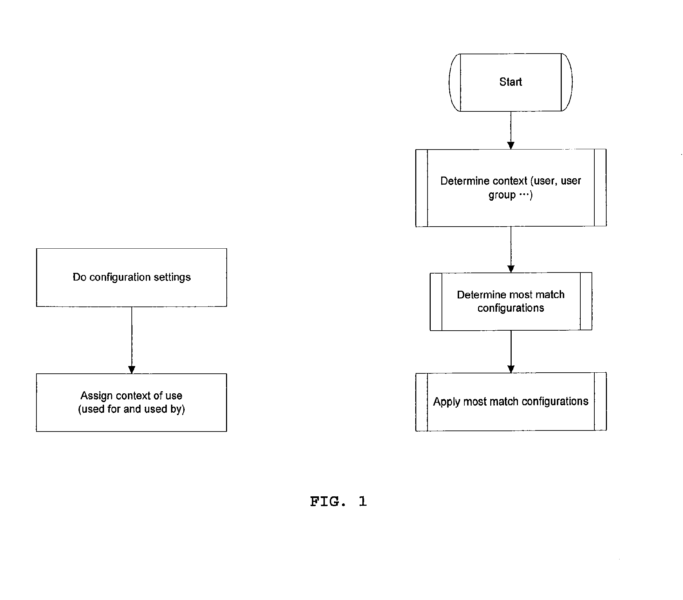

The method of the present invention is illustrated by the flow chart of figure 1.

According to the present invention first a configuration set up is performed. During this set up different sets of configuration values for parameters of the software application are generated for different cases. For example in the case of software for generating reports, sets of configuration values are determined for a number of different lay-outs of reports.

Then the so-called 'context of use' is assigned to the different sets of configuration values. 'Context of use' is in the present invention, defined as at least one item or code which identifies runtime context. In a hospital environment it can be a user (e.g. Doctor X) or a type of user (e.g. a radiologist), a user group, a department, an identification of the workstation on which the application is used etc.

In one example a different code is assigned for 'used by' and 'used for' level. So by a simple sorting on used by and used for level, the most matching configuration can be selected.

Suppose there are 4 'used by' levels: all procedures, modality type, body part and procedure definition. Procedure definition is the most specific and gets highest priority score, all procedures is the most generic and gets lowest priority score. An integer code can be assigned to the different levels, based on priority level. E.g. procedure definition: 100, body part: 80, modality type: 40, all procedures: 20. Then for each configuration entity, a level code can be assigned. A hanging protocol for procedure definition = "CT Head" gets level code as 100, and a hanging procedure for body part = "chest" gets level code as 80. When the system tries to find the most match hanging protocol, it adapts an algorithm, to get all the applicable hanging protocols matching the context, and then sorting them by the level code descending, then the first one will b the most matching one.

Context of use is composed of different levels which each get a different associated priority score.

A set of configuration values for a configuration which is most specific is preferably given the highest priority score. A set of configuration values for a configuration which is generic and would fit most cases is given the lowest priority score.

Next, other configuration value sets are generated for other configuration levels to which lower priority scores are assigned.

All these configuration sets together with the associated context and the priority assignment are stored in a data repository such as a database, accessible to the software application.

During runtime of the software application the runtime context is first determined. As mentioned earlier, in the described example runtime context may comprise one or more of the following items: the user login, the department the user belongs to, the facility the user is working in, the user group or department, the identification of the workstation the user is using, the group to which this workstation belongs, the request procedure or study he is doing, the modality type, the body part being examined etc.

Next, the software application applies the priority algorithm searches in the data repository the configuration values set which matches the run time context. If such a configuration values set is found, the settings of this configuration are applied. However, if the configuration matching the run time context is not found, the selection of configuration values to be applied depends on the level of the run time context. If this is already the level with the highest score, the system default settings will be applied. It this is not the level with the highest score, a configuration values set will be selected matching a level with a higher associated score than the score of the current level. The hierarchy of the configuration levels ensures that the most relevant configuration value set according to the runtime context is selected and applied.

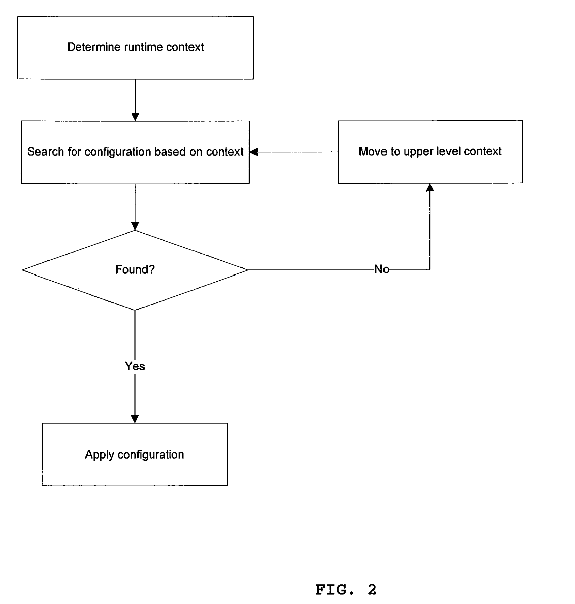

This procedure is illustrated in figure 2.

An example is described below.

The example relates to text macro's to be used by radiologists when generating a report on a study.

Suppose that two text macro's are configured, one named 'macro1' and the other named 'macro2'. Macro 1 has the context of use: procedure definition being CT (computed tomography) head, and user being Dr. Peter. Macro2 has as context of use: procedure definition being CT head and user being 'system' (meaning that this is a system level, all users can use it).

When doctor Peter is reporting on a CT Head study, text macro named 'macro1' will be the best match, whereas for doctor Paul macro2 will be the best match. If doctor Peter is reporting on a CT ankle, Macro1 will not be the best match so macro2 will be selected for his report.

The invention is implemented by running an artificial intelligence algorithm in the application's runtime.

First all possible configuration levels are abstracted and prioritized based on actual usage. Then to each configuration level a level code is assigned to reflect their priority score. When doing configuration, each configuration set is stored together with the level code in a central repository. During the runtime, the artificial intelligence algorithm will access the configuration repository, fetch all applicable configuration sets according to environmental contexts, sorting them by priority score and get the most matching one.

Having described in detail preferred embodiments of the current invention, it will now be apparent to those skilled in the art that numerous modifications can be made therein without departing from the scope of the invention as defined in the appending claims.

## Claims

1. A method of configuring a software application comprising the steps of
- identifying different levels of context of use of said software application and associating a priority score with each of said levels,
- generating and storing in a data repository configuration values sets for said software application for instances and levels of said context of use,
- during run-time of said software application, determining runtime context of use with associated priority score,
- retrieving from said data repository a configuration values set which matches the level of said runtime context of use,
- if a matching configuration values set is found, applying this set,
- if it not found, searching for a configuration values set corresponding to a level of context of use with a higher associated priority score and applying this configuration values set.

2. A method according to claim 1 wherein said software application is a software application running in a medical environment.

3. A computer program product adapted to carry out the method of any of the preceding claims when run on a computer.

4. A computer readable medium comprising computer executable program code adapted to carry out the steps of any of claims 1 - 2.
